## Europäisches Patentamt
## European Patent Office
## Office européen des brevets

(19)

(11) Publication number: **0 263 049**
A2

# EUROPEAN PATENT APPLICATION

(12)

(21) Application number: **87500044.0**

(22) Date of filing: **22.06.87**

(51) Int. Cl.⁴: **F 22 B 1/28**
F 22 B 37/26, A 61 M 16/16

(30) Priority: **23.06.86 ES 556473**

(43) Date of publication of application:
**06.04.88 Bulletin 88/14**

(84) Designated Contracting States:
**AT BE CH DE FR GB GR IT LI LU NL SE**

(71) Applicant: **S.O.R. INTERNACIONAL, S.A.**
**Polig. Ind. Can Casablancas Empordà i Moianè s/n**
**E-08192 Sant Quirze Del Vallès (ES)**

(72) Inventor: **Sanchez Soriano, Manuel**
**Polig. Ind. Can. Casablanca Empordà i Moianès s/no**
**E-08192 Sant Quirze del Vallès Barcelona (ES)**

(74) Representative: **Manresa Val, Manuel**
**Gerona n. 34**
**E-08010 Barcelona (ES)**

(54) **Improvements made in a device to achieve water ebulliency.**

(57) These improvements are directed to provide said device with two tanks, (1,4), one within the other, and both designed to be water filled (N1) without dousing the inside tank (4). Said tank (4) is top opened (5) and provided with a heating element (7) to achieve water ebulliency. The outside tank (1) has a delivery (2) wherefrom only the evaporated water (Ve) goes out while the engendered foam-water (Vp) is condensed and comes back to the outside tank (1). At the bottom of the inside tank (1) a hole (6) is provided with a section (d) which rate of water inflow (Vi) is equivalent to the evaporated water rate (Ve) plus the foam-water rate (Vp). The outside tank (1) has an inlet (3) to refill water therefrom when level reaches a minimum (N2).
Applicable to any water diffusers in aesthetics and medicine.

Bundesdruckerei Berlin

## Description

Improvements made in a device to achieve water ebulliency.

The present invention patent refers to the improvement made in a device to achieve water ebulliency in the performance and diffusion of steam. In fact, the various applications used in the steam diffusion are well known and its application in aesthetics and medicine should be remarked among them.

One of the problems raised in the water heaters designed to achieve said purposes is seen in that the foam engendered during the phenomenon of water ebulliency inconveniently goes out from the heater opening, i.e. is outwardly "spitted" with remarkably nuisance for the user taking up those vapours, the more so if the temperature of said foam is realised.

Many tests have been performed so far to evade these drawbacks but all them proved mediocre. Conversely, the applicant has found a satisfactory solution to these drawbacks with the present improvements essentially directed to provide the respective device with two tanks, one outside and the other inside, and have them water filled up to a certain level so that the tank insides are not doused. The inside tank is provided with a heating element whereon water ebulliency is caused and is additionally provided with an opening at the upper side wherefrom foam and steam actually go out; only said steam is outwardly released through one delivery of the outside tank while the foam on finding out a lower temperature in the outside tank is again converted into water and fed back to said outside tank. The inside tank is on the other hand provided with a hole at the bottom and with a lower section than that of the upper opening and by means of this hole, the water rate, eventually converted into foam is automatically being refilled and also the water rate converted into steam outwardly released, the production of foam is thus consistently limited.

To make this disclosure more understandable, a drawing sheet is attached to the present specification wherein has been shown a practical case of embodiment cited only by way of a non-exhaustive example of the scope of the present invention patent.

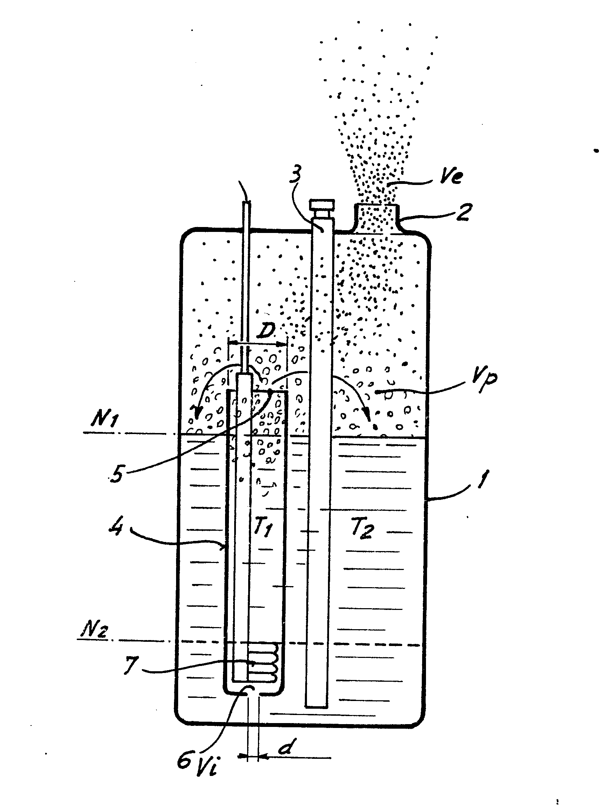

In the figure can be noted: an outside tank -1- provided with a steam delivery -2- and an inlet conduit -3- for water filling; and an inside tank -4- separated from above, from below and sideways from the outside tank -1- and showing at the uppermost side an opening -5- and a hole -6- at the bottom of a lower section -d- than that of the section -D- of the opening -5-. Also said inside tank -4- is internally provided with a heating element -7- such as a cladded electrical resistor.

The operation of the device according to these present improvements is to fill the outside tank -1- with water and through vessels in communication and inside -4- also to a level -N1- wherefrom the latter is not doused. With the performance of the heating element -7- the water remaining in the inside tank -4- comes in ebulliency, a certain amount of foam is formed (according to the kind of water, salts and other intrinsic components of the water) ans steam is released. The outside tank -1- due to external influences is logically at a temperature -T2- lower than a temperature -T1- of the inside tank -4- so the bubbles of said foam, before delivery -2- is reached, are condensed and again converted into water which is added to the water already in the outside tank -1-; only steam is outwardly released by delivery -2- while bubbles as already said do not reach outside and do not consequently trouble the user.

Another basic characteristic of the device according to the present improvements is seen in that the amount of foam engendered is automatically adjusted as the water provision is limited by said hole -6-. In fact, said hole must be dimensioned so that the hole section meets the equation:

$$Vi = Ve + Vp$$

where -Vi- is the inflow rate of water through the same hole -6-, -Ve- is the foam-water rate and -Vp- is the evaporated water rate, i.e., the amount of water inflow from the hole will always be equivalent to the addition of foam-water plus evaporated water. The amount of foam-water engendered is thus stabilized since the latter depends and is consistently adjusted by the water fed through the section of said bottom hole -6-.

Briefly, with the device according to the present improvements the outwardly release of foam-water is avoided from one part and production of said foam is consistently stabilized from another part.

The outside tank -1- retrieves the foam-water rate -Vp- of the condensed bubbles again converted into water as previously said; the evaporated water or outwardly released steam obviously is not retrieved, so the water level comes down to a minimum -N2- at which time water shall be added from outside in a way known such as for example through a conduit -3-.

## Claims

1.- Improvements made in a device to achieve water ebulliency, particularly applicable to steam diffusers for aesthetics and medicine, characterized in that said device is provided with two tanks, one within the other, in a way such as the inside tank is separated from above, from below and sideways from the outside tank; said outside tank has an upper delivery while the inside tank is top opened, and has a hole at its bottom which communicates with the outside tank, and is provided with a conventional heating element such as a cladded electrical resistor, all that functionally arranged to fill both water tanks without dousing the inside tank and in that on being ebulliency caused in said inside tank by means of said heating element, the evaporated water is released through said upper delivery of the outside tank while the foam-water engendered, before said delivery is reached, is condensed and comes back to the outside tank with the peculiarity that the hole at the bottom of the inside tank has a dimension such as its water inflow rate is equivalent to said evaporated water rate plus the foam-water rate, a sealing inlet in the outside tank has also been provided to refill water when is required by the level of said outside tank.

0263049